# EUROPEAN PATENT APPLICATION

(11) **EP 1 097 723 A2**
(43) Date of publication of application: **09.05.2001**
(21) Application number: 00500216.7
(22) Date of filing: 24.10.2000
(51) Int. Cl.: A61L 9/03, B60H 3/00

(54) **Electric air-freshener for motor vehicles**

(30) Priority: 04.11.1999 ES 9902786 U
(71) Applicant: Arias Vidal, Maria José, 11540 Sanlucar de Barrameda, Cadiz (ES)
(72) Inventor: Arias Vidal, Maria José, 11540 Sanlucar de Barrameda, Cadiz (ES)
(74) Representative: Maldonado Jordan, Julia

(57) **Abstract**

This invention relates to an air-freshener for use in motor vehicles. It is electrically-operated, drawing current generated by the vehicle itself (12V), providing the known electric air-freshener with a new structure to make it suitable for use in motor vehicles, characterised by consisting basically of two distinct parts: one being the power plug to plug into the cigarette lighter socket in the vehicle and operate from its 12V supply; the other holding the perfume bottle, housed in both and holding the resistance element in whose central hole the wick of the threaded perfume bottle is inserted in the part shown in figure 12.

## Description

### PURPOSE OF THE INVENTION

This invention is an air-freshener for use in motor vehicles. It is electrically-operated, drawing current generated by the vehicle itself (12V).

### ANTECEDENTS OF THE INVENTION

There exist various types of air-fresheners for vehicles, consisting of figures impregnated with perfume which are hung inside the vehicle or placed in front of air intake grilles.
There also exist electric air-fresheners for use in the home which are powered by the mains electricity supply (220V).

### DESCRIPTION OF THE INVENTION

The purpose of the invention is to provide the known electric air-freshener with a new structure to make it suitable for use in motor vehicles.
This involves replacing the plug used with a 220V electricity supply by a plug which accepts the 12V supply provided by the vehicle, more specifically from the cigarette lighter socket.
The electrical current heats a resistance element in contact with a metal plate surrounding the housing for the perfume-impregnated wick, which in turn is contained in a small threaded bottle and fastened to the casing. This casing fits via snugs inside another casing which contains the resistance element. The invention works by the resistance element transmitting its heat to the metal plate which causes the liquid perfume to evaporate, leaving its characteristic scent in the surrounding air.
The resistance is of the "chalk" type, of 8 watts and 47 ohms.

### BRIEF DESCRIPTION OF DRAWINGS

Figure 1: Shows a side view of the vehicle electric air-freshener power plug.

Figure 2: Shows a plan view of the vehicle air-freshener power plug. Figure 3: Shows one of the component parts of this power plug, the central slot of which houses the negative terminal (fig. 4).

Figure 4. Shows the negative terminal of the plug, which is a piece of metal with the wire soldered to its end.

Figure 5: Shows the positive terminal which is coupled to the screw thread shown in figure 3.

Figure 6: Shows a plan view of the plastic part which houses the resistance element and the metal part. This plastic part has a central hole.

Figure 7: Shows the bottom view of the part described above.

Figure 8: Shows the side view of the same part.

Figure 9: Shows the bottom view of the electric air-freshener, with the plastic part described in figure 6 housed inside it.

Figure 10: Shows the side view of the air-freshener without the inside part where the perfume bottle is housed and secured by a screw thread. The figure also shows the inner guide which slides into the power socket.

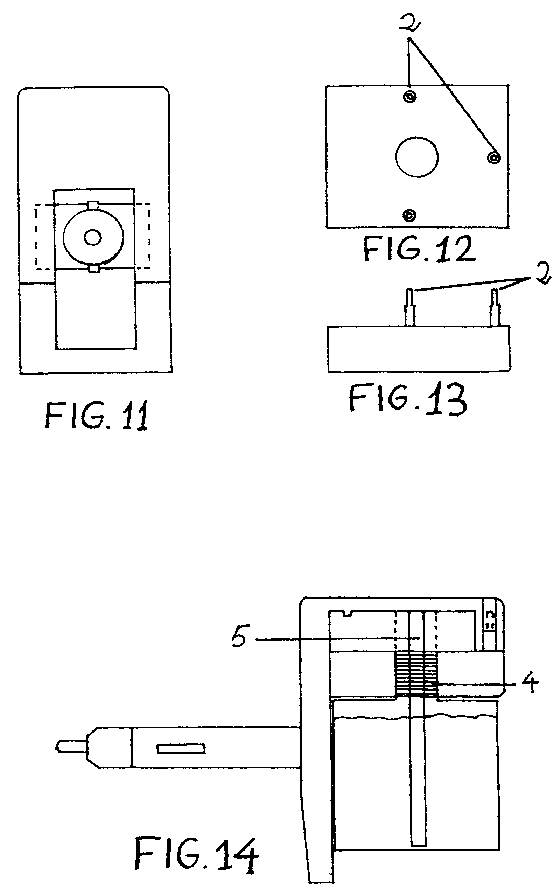

Figure 11: Shows the front view of the air-freshener, including the plug section and inner slot along which it can slide.

Figure 12: Shows the plan view of the bottom plastic part, which is fastened to the other by snugs and which uses a screw thread to house the perfume bottle.

Figure 13: Shows the side view of the part described above.

Figure 14: Shows the complete air-freshener with the perfume bottle fitted, including the part to which it is fastened and where the wick is housed in the top part.

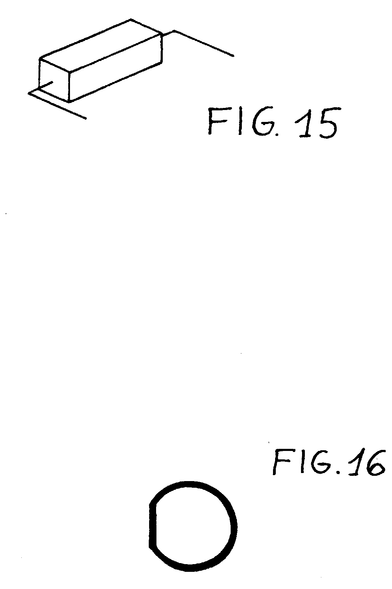

Figure 15: Shows the resistance element in "chalk" form of 8 watts and 47 ohms.

Figure 16: Shows the metal part which is in contact with and heated by the resistance element.

### DESCRIPTION OF A PREFERRED REALISATION

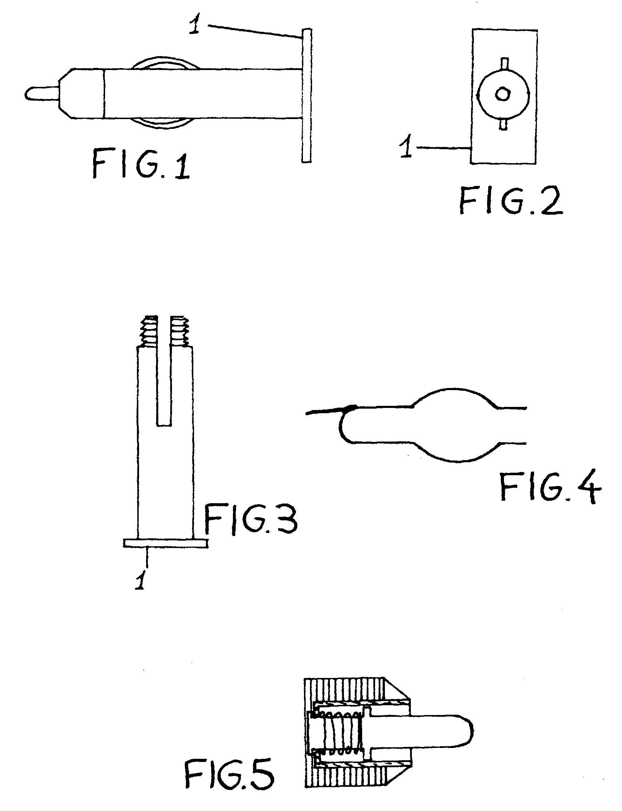

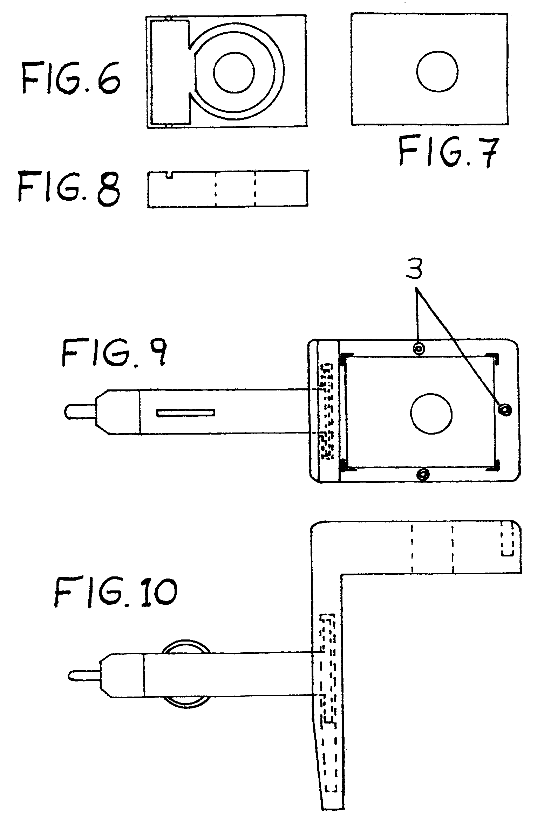

Figures 3, 4 and 5, fitted together form a single unit as shown in figures 1 and 2 and which constitute a device to pick up current. This is achieved via a semirigid rubber stuck at the back and inserted in the guide formed by the inside of the casing with an up and down sliding movement. Preferred materials used for manufacture are plastic capable of withstanding the temperature produced by the resistance element when it is connected to the electrical supply, except in the case of the metal parts which must conduct heat (figure 16) and the electrical wiring which must be specially insulated to prevent it becoming too hot.

## Claims

1. Electric air-freshener for motor vehicles, characterised by consisting basically of two distinct parts: one being the power plug (fig. 1 ) to plug into the cigarette lighter socket in the vehicle and operate from its 12V supply; the other holding the perfume bottle (figs. 10 and 12), housed in both and holding the resistance element (fig. 6) in whose central hole the wick of the threaded perfume bottle is inserted in the part shown in figure 12.

2. Electric air-freshener for motor vehicles, characterised by having a metal plate (fig. 16) contained in a plastic part (fig. 6), in the central hole of which the perfume-impregnated wick is housed. This wick receives the heat produced by the resistance element (fig. 15) and makes the perfume evaporate.

3. Electric air-freshener for motor vehicles, characterised by having a "CHALK" type resistance (fig. 15) of forty-seven ohms and eight watts.
